# EUROPÄISCHE PATENTSCHRIFT

(11) **EP 2 550 062 B1**
(45) Veröffentlichungstag und Bekanntmachung des Hinweises auf die Patenterteilung: **16.05.2018**
(21) Anmeldenummer: 11710690.6
(22) Anmeldetag: 15.03.2011
(51) Int. Cl.: A61N 5/10

(54) **VERFAHREN ZUM BETREIBEN EINER BESTRAHLUNGSVORRICHTUNG**
METHODS FOR OPERATING A RADIATION DEVICE
PROCÉDÉS POUR FAIRE FONCTIONNER UN DISPOSITIF D'IRRADIATION

(30) Priorität: 25.03.2010 DE 102010012654; 20.05.2010 DE 102010021119
(43) Veröffentlichungstag der Anmeldung: 30.01.2013
(73) Patentinhaber: GSI Helmholtzzentrum für Schwerionenforschung GmbH, 64291 Darmstadt (DE)
(72) Erfinder: BERT, Christoph, 63741 Aschaffenburg (DE); RIETZEL, Eike, 64331 Weiterstadt (DE); SAITO, Nami, 64291 Darmstadt (DE)
(74) Vertreter: Mergel, Volker
(86) Internationale Anmeldenummer: PCT/EP2011/001266
(87) Internationale Veröffentlichungsnummer: WO 2011/116889

(56) Entgegenhaltungen:
- EP-A1- 1 477 206
- EP-A1- 1 656 966
- US-A1- 2008 159 478
- SAWANT A ET AL: "Toward Submillimeter Accuracy in the Management of Intrafraction Motion: The Integration of Real-Time Internal Position Monitoring and Multileaf Collimator Target Tracking", INTERNATIONAL JOURNAL OF RADIATION: ONCOLOGY BIOLOGY PHYSICS, PERGAMON PRESS, USA, Bd. 74, Nr. 2, 1. Juni 2009 (2009-06-01), Seiten 575-582, XP026094995, ISSN: 0360-3016, DOI: DOI:10.1016/J.IJROBP.2008.12.057 [gefunden am 2009-03-26]
- QING REN ET AL: "Adaptive prediction of respiratory motion for motion compensation radiotherapy", PHYSICS IN MEDICINE AND BIOLOGY, TAYLOR AND FRANCIS LTD. LONDON, GB, Bd. 52, Nr. 22, 21. November 2007 (2007-11-21), Seiten 6651-6661, XP020127250, ISSN: 0031-9155
- GREGORY C SHARP ET AL: "Prediction of respiratory tumour motion for real-time image-guided radiotherapy; Prediction of respiratory tumour motion", PHYSICS IN MEDICINE AND BIOLOGY, TAYLOR AND FRANCIS LTD. LONDON, GB, Bd. 49, Nr. 3, 7. Februar 2004 (2004-02-07), Seiten 425-440, XP020024002, ISSN: 0031-9155, DOI: DOI:10.1088/0031-9155/49/3/006
- BERT CHRISTOPH ET AL: "4D treatment planning for scanned ion beams", RADIATION ONCOLOGY, BIOMED CENTRAL LTD, LO, vol. 2, no. 1, 3 July 2007 (2007-07-03), page 24, XP021030775, ISSN: 1748-717X, DOI: 10.1186/1748-717X-2-24

## Beschreibung

Die Erfindung ist in den unten anhängenden Ansprüchen definiert.

Die Erfindung betrifft ein Verfahren zum Betreiben einer Bestrahlungsvorrichtung zur Bestrahlung zumindest eines Zielvolumenbereichs eines Zielkörpers, wobei der zu bestrahlende Zielvolumenbereich des Zielkörpers durch eine Steuereingabe der Bestrahlungsvorrichtung verändert werden kann. Weiterhin betrifft die Erfindung eine Bestrahlungsvorrichtung zur Bestrahlung zumindest eines Zielvolumenbereichs eines Zielkörpers, die zumindest eine Bestrahlungs-Anpassvorrichtung aufweist.

Zwischenzeitlich wird in unterschiedlichsten Bereichen der Technik eine Bestrahlung von Gegenständen durchgeführt. Je nach konkretem Einsatzerfordernis werden hierbei unterschiedliche Bestrahlungsverfahren sowie verschiedene Strahlungsarten verwendet. So ist es in manchen Gebieten der Technik erforderlich, Gegenstände flächig bzw. räumlich und hierbei möglichst gleichmäßig zu bestrahlen. Dies ist beispielsweise der Fall, wenn Materialien gehärtet oder sonst wie bezüglich ihrer Materialeigenschaften verän-dert werden sollen. Auch ist es zwischenzeitlich beispielsweise auf dem Gebiet der Lebensmitteltechnik üblich geworden, Lebensmittel unter Verwendung bestimmter Strahlungsarten haltbar zu machen.

In anderen Gebieten der Technik ist es dagegen erforderlich, bestimmte Teilbereiche des zu bestrahlenden Gegenstands mit einer bestimmten, typischerweise besonders hohen Dosis zu bestrahlen, während die übrigen Teile des Gegenstandes nicht bzw. so wenig wie möglich bestrahlt werden sollten. Ein Beispiel hierfür ist die Strukturierung von Mikroprozessoren oder sonstigen Mikrostrukturen bzw. Nanostrukturen unter Verwendung von elektromagnetischer Strahlung (zum Teil bis in den Röntgenbereich hinein) sowie bildgebender Masken.

Die Strukturierung der Dosisverteilung erfolgt dabei nicht unbedingt nur zweidimensional, sondern kann vielmehr auch in allen drei Raumrichtungen erfolgen. Dadurch ist es möglich, einen Volumenbereich, der im Inneren eines zu bestrahlenden Körpers liegt, direkt und unmittelbar zu bestrahlen, ohne den Körper (insbesondere dessen Außenhülle) beschädigen bzw. öffnen zu müssen. Dabei kann der zu bestrahlende Körper (insbesondere ein im Inneren des zu bestrahlenden Körpers liegender zu bestrahlender Volumenbereich) nicht nur statisch/unbewegt vorliegen. Vielmehr kann es auch vorkommen, dass sich der Zielkörper bzw. Teile des Zielkörpers (insbesondere der zu bestrahlende Volumenbereich) bewegen. Eine Bewegung kann nicht nur translatorisch relativ zu einem äußeren Koordinatensystem erfolgen, sondern auch in Form einer Verschiebung verschiedener Bereiche des zu bestrahlenden Körpers relativ zueinander (einschließlich Verdrehungen und Verformungen) erfolgen.

Um derartige, (in sich) bewegte Körper bestrahlen zu können, werden sogenannte vierdimensionale Bestrahlungsverfahren verwendet. Dabei handelt es sich schlussendlich um dreidimensionale Bestrahlungsverfahren, die eine zeitliche Variation aufweisen (mit der Zeit als vierter Dimension). Beispiele für derartige Materialbearbeitungsverfahren gibt es im Bereich der Materialwissenschaften bei der Herstellung hochintegrierter Bauteile (insbesondere Mikroprozessoren und Speicherchips) sowie bei der Herstellung von mikrostrukturierten und nanostrukturierten Mechaniken.

Ein weiteres Gebiet der Technik, bei dem derartige dreidimensionale bzw. vierdimensionale Bestrahlungsverfahren eingesetzt werden, liegt im Bereich der Medizintechnik. Hier ist es in der Regel ebenfalls erforderlich, bestimmte Volumenbereiche innerhalb eines Körpers, wie beispielsweise Tumore, mit einer möglichst hohen Dosis zu beaufschlagen, wohingegen das umliegende Gewebe möglichst wenig bzw. vorzugsweise im Wesentlichen überhaupt nicht mit einer Dosis belastet werden sollte. Dies gilt in besonderem Maße, wenn es sich bei dem umgebenden Gewebe um ein sogenanntes kritisches Gewebe handelt, wie beispielsweise um ein empfindliches Organ (fachsprachlich als OAR für "Organ At Risk" bezeichnet) handelt. Hierbei kann es sich beispielsweise um das Rückenmark, um Blutgefäße oder Nervenknoten handeln.

Gerade bei der Bestrahlung von bewegten Zielvolumina treten vielfältige, zum Teil noch nicht bzw. nicht befriedigend gelöste Probleme auf. Grundsätzlich gibt es eine große Anzahl an Lösungsmöglichkeiten. Speziell für die Verwendung mit Scanning-Verfahren werden insbesondere drei spezielle Herangehensweisen diskutiert. Hierbei handelt es sich um sogenannte Rescanning-Verfahren, Gating-Verfahren sowie Tracking-Verfahren.

Bei Rescanning-Verfahren wird der zu bestrahlende Körper mit einer großen Anzahl von hintereinander erfolgenden Bestrahlungsvorgängen belegt. Bei einem zyklisch wiederkehrenden Bewegungsmuster des bewegten Körpers (bzw. des zu bestrahlenden Zielgebiets) erfolgt dadurch im statistischen Mittel eine ausreichend starke Bestrahlung des Zielvolumens. Problematisch ist jedoch, dass es fast unvermeidbar zu einer relativ hohen Strahlenbelastung von an sich nicht zu bestrahlenden Teilbereichen des Zielkörpers kommt. Darüber hinaus eignen sich Rescanning-Verfahren prinzipbedingt vornehmlich für relativ schnell verlaufende, zyklisch wiederkehrende Bewegungen.

Bei Gating-Verfahren erfolgt eine aktive Bestrahlung des Zielkörpers nur dann, wenn sich der zu bestrahlende Volumenbereich in einem relativ eng begrenzten, definierten Gebiet befindet. Zu anderen Zeitpunkten erfolgt dagegen keine Bestrahlung (in der Regel durch Abschaltung der Teilchenstrahls). Grundsätzlich liefern Gating-Verfahren gute Bestrahlungsergebnisse. Nachteilig ist jedoch die längere Bestrahlungsdauer, die unter anderem zu höheren Kosten führt.

Einen besonders vielversprechenden Ansatz stellen die Tracking-Verfahren dar. Hierbei wird der Bereich, in dem die Strahlung einwirkt, korrespondierend zur Bewegung des zu bestrahlende Volumenbereichs des Zielkörpers nachgeführt. Tracking-Verfahren vereinen die Vorteile einer präzisen, zielgenauen Behandlung mit relativ kurzen Bestrahlungszeiten.

Obgleich Tracking-Verfahren grundsätzlich funktionieren, besteht nach wie vor die Notwendigkeit, diese zu verbessern. Insbesondere ist die Genauigkeit der Bestrahlung (einschließlich der Bestrahlungseinwirkung, des Dosiseintrags usw.) gegenüber den heutzutage erzielbaren Werten nach wie vor verbesserungswürdig.

Es ist die Aufgabe der vorliegenden Erfindung ein verbessertes Verfahren und eine verbesserte Vorrichtung zur Bestrahlung eines Zielvolumens zu schaffen.

Die Aufgabe wird mit den Merkmalen der unabhängigen Ansprüche gelöst. Vorteilhafte Weiterbildungen des Verfahrens und der Vorrichtung ergeben sich aus den unabhängigen Ansprüchen.

Es wird ein Verfahren zum Betreiben einer Bestrahlungsvorrichtung zur Bestrahlung zumindest eines Zielvolumenbereichs eines Zielkörpers vorgeschlagen, bei dem der zu bestrahlende Zielvolumenbereich des Zielkörpers durch eine Steuereingabe der Bestrahlungsvorrichtung verändert werden kann, wobei bei der Erzeugung der Steuereingabe zumindest eine zwischen einer Steuereingabe und einer Reaktion der Bestrahlungsvorrichtung liegende Verzögerungszeitspanne zumindest zeitweise und/oder zumindest bereichsweise berücksichtigt wird. Die Erfinder haben festgestellt, dass bei der Ansteuerung der Bestrahlungsvorrichtung bislang erstaunlicherweise vernachlässigt wurde, dass zwischen dem "Anlegen" einer Steuereingabe an die Bestrahlungsvorrichtung und der effektiven Reaktion der Bestrahlungsvorrichtung auf die Steuereingabe (also der Freisetzung der Strahlung in das angesteuerte Gebiet hinein) zum Teil verblüffend lange Zeitintervalle liegen können. Diese liegen in einer Größenordnung, die im Wesentlichen mit der Größenordnung der Bestrahlungsdauer pro Rasterpunkt und/oder der Grö-βenordnung der Bewegungsgeschwindigkeit des Zielvolumenbereichs übereinstimmt. Wenn also beispielsweise bei der Bestrahlung eines bewegten Zielvolumenbereichs eine Erfassungsvorrichtung den Zielvolumenbereich lokalisiert hat und der so erfasste Zielvolumenbereich durch eine entsprechende Steuereingabe angesteuert wird, so kann es ohne weiteres vorkommen, dass zu dem Zeitpunkt, zu dem die Bestrahlungsvorrichtung die Strahlung im Zielvolumenbereich zur Verfügung stellt, sich der Zielvolumenbereich bereits weiter bewegt hat. Dadurch kann es vorkommen, dass der Zielvolumenbereich nicht mehr (ausreichend) genau mit der Strahlung beaufschlagt wird. In einer durchaus nicht zu vernachlässigenden Anzahl von Fällen tritt auch der Effekt auf, dass sich der Zielvolumenbereich in Folge seiner Bewegung bereits außerhalb des schlussendlich bestrahlten Volumenbereichs befindet. Auch eine Veränderung der Bestrahlungsintensität, beispielsweise während einer Behandlung oder von Behandlungstag zu Behandlungstag (die beispielsweise durch eine Veränderung in der Strahlungsintensität der Strahlungsquelle der Bestrahlungsvorrichtung hervorgerufen werden kann) kann zu insbesondere schwankenden Zeitverzögerungen vom Ansteuern bis zum "Anfahren" eines Rasterpunkts führen. Mit anderen Worten kann der Zeitpunkt, zu dem ein noch zu bestrahlender Rasterpunkt von der Bestrahlungsvorrichtung mit einer bestimmten Dosis belegt wird, in Abhängigkeit von der zwischen dem aktuellen Zeitpunkt und dem Zeitpunkt der Beaufschlagung des Rasterpunkts liegenden Bestrahlungsintensität durch die Bestrahlungsvorrichtung gegebenenfalls stark schwanken. Wenn man bei der Erstellung der Steuereingabe (wobei die Steuereingabe aus einem oder aus mehreren Werten bestehen kann) diese Verzögerungszeitspanne (bzw. mehrere Verzögerungszeitspannen) berücksichtigt, kann daher die Genauigkeit der Bestrahlung zum Teil deutlich vergrößert werden. Besonders ausgeprägt ist der Effekt bei relativ kleinen und genau zu bestrahlenden Zielvolumenbereichen, bei sich relativ schnell bewegenden Zielvolumenbereichen und/oder bei relativ stark schwankenden Bestrahlungsintensitäten (zum Beispiel Teilchenfluss bei Teilchenbeschleunigern).

Vorteilhaft ist es, wenn bei dem Verfahren eine Mehrzahl von Verzögerungszeitspannen berücksichtigt wird, insbesondere eine Mehrzahl von Verzögerungszeitspannen mit zumindest zeitweise und/oder zumindest teilweise unterschiedlicher und/oder variierender Länge. Insbesondere ist es vorteilhaft, wenn möglichst sämtliche Verzögerungszeitspannen berücksichtigt werden, die in Relation zur Größe des Zielvolumenbereichs, zur Größe des jeweils aktiven Bestrahlungsbereichs, zur Geschwindigkeit der Bewegung des Zielvolumenbereichs und/oder zur aktuellen Bestrahlungsintensität eine relativ lange Zeitdauer beziehungsweise eine relativ starke Schwankung der Zeitdauer aufweisen, derart, dass sich die Berücksichtigung der entsprechenden Verzögerungszeitspanne (bzw. Verzögerungszeitspannen) gegebenenfalls signifikant bemerkbar macht. Werden (im Wesentlichen) alle relevanten Zeitverzögerungen betrachtet, so kann dies in einer besonders vorteilhaften und genauen Bestrahlung des Zielvolumenbereichs beziehungsweise des Zielkörpers resultieren. Insbesondere haben die Erfinder erkannt, dass Verzögerungszeitspannen mit einer unterschiedlichen Länge auftreten können, und vorzugsweise insbesondere bezüglich ihrer unterschiedlichen Länge berücksichtigt werden sollten. So ist es möglich, dass die Änderung eines ersten Parameters der Bestrahlung eine andere Verzögerungszeitspanne mit sich bringt, als dies bei der Änderung eines zweiten Parameters der Bestrahlung der Fall ist. Ergänzend sollte darauf hingewiesen werden, dass die Verzögerungszeitspannen sowohl (zum Teil) hintereinander liegen können ("sequenziell"), und dementsprechend addiert werden sollten, aber auch (zum Teil) "parallel" zueinander liegen können, und dementsprechend unabhängig voneinander betrachtet werden sollten. Eine derartige "parallele" Lage der Verzögerungszeitspannen kann insbesondere dann vorliegen, wenn bei der Verstellung voneinander unabhängiger Parameter der Bestrahlung zeitliche Verzögerungen auftreten.

Insbesondere hat es sich als vorteilhaft erwiesen, wenn bei dem Verfahren zumindest eine Verzögerungszeitspanne berücksichtigt wird, die bei einer Bestrahlungsanpassung in zumindest einer lateral zur Bestrahlungsrichtung liegenden Richtung auftritt und/oder zumindest eine Verzögerungszeitspanne berücksichtigt wird, die in einer longitudinal zur Bestrahlungsrichtung liegenden Richtung auftritt. So haben die Erfinder festgestellt, dass eine laterale Ablenkung (beispielsweise durch Anlegen eines entsprechenden Stroms an geeignet angeordnete Magnetfeldspulen) zu relativ kleinen Verzögerungszeitspannen führt. Eine Anpassung einer Bestrahlung in einer Richtung, die longitudinal zur Bestrahlungsrichtung verläuft, ist dagegen in aller Regel deutlich zeitaufwändiger. Die Zeitdauern können dabei um den Faktor 10 oder mehr auseinander liegen. Wenn bei der Erstellung der Steuereingabe auf diese beiden unterschiedlichen (gegebenenfalls zusätzlich oder alternativ auch weitere) Charakteristika eingegangen wird, so kann eine besonders hochqualitative und genaue Bestrahlung des Zielvolumenbereichs bzw. des Zielkörpers erfolgen.

Besonders sinnvoll kann es speziell in diesem Zusammenhang sein, wenn zumindest eine Verzögerungszeitspanne bei einer Bestrahlungsanpassung in zumindest einer lateral zur Bestrahlungsrichtung liegenden Richtung zumindest zeitweise und/oder zumindest teilweise in einem Bereich unter 250 ms, insbesondere einem Bereich unter 100 ms, bevorzugt in einem Bereich unter 20 ms, besonders bevorzugt in einem Bereich unter 1 ms liegt und/oder wenn zumindest eine Verzögerungszeitspanne einer Bestrahlungsanpassung in einer longitudinal zur Bestrahlungsrichtung liegenden Richtung zumindest zeitweise und/oder zumindest teilweise in einem Bereich unter 150 ms, insbesondere in einem Bereich unter 80 ms, bevorzugt in einem Bereich unter 40 ms, besonders bevorzugt in einem Bereich zwischen 20 und 30 ms liegt. Die genannten Werte haben sich insbesondere für Teilchenstrahlen mit Hadronen und/oder im Zusammenhang mit Scanning-Verfahren (einschließlich Raster-Scanning-Verfahren sowie intensitätsgesteuerte Raster-Scanning-Verfahren) als besonders vorteilhaft erwiesen. Insbesondere sind die genannten Werte von Vorteil, wenn eine Richtungsanpassung in lateraler Richtung durch ein (oder mehrere) senkrecht zueinander stehendes Magnetfeldspulenpaar erfolgt und/oder eine Bestrahlungsanpassung in einer longitudinal zur Bestrahlungsrichtung liegenden Richtung durch passive, bewegte Energiemodulatorelemente erfolgt. Insbesondere kann es sich bei den passiven, bewegten Energiemodulatorelementen um einen keilförmigen Körper aus einem energieabsorbierenden Material handeln, der mit Hilfe eines Motors, insbesondere eines Schrittmotors, derart bewegt wird, dass die Länge der Strecke, die die Strahlung (beispielsweise ein Teilchenstrahl) durch den Körper hindurch zurücklegen muss, durch die Verschiebung des energieabsorbierenden Körpers verändert werden kann. Oftmals wird auch ein Paar aus zwei keilförmigen Körpern verwendet, die punktsymmetrisch zueinander angeordnet sind und "gegeneinander" bewegt werden. Zum Teil wird zwischenzeitlich auch eine größere Anzahl von derartigen Körpern vorgeschlagen. Eine weitere Variation des zu Grunde liegenden Gedankens besteht darin, dass anstelle des Körpers der Teilchenstrahl selbst relativ zu den Körpern bewegt wird, wodurch ein ähnlich gearteter Effekt erzielt werden kann.

Weiterhin wird vorgeschlagen, dass das Verfahren derart durchgeführt wird, dass zumindest eine Verzögerungszeitspanne Signalaufbereitungseffekte und/oder Signalweiterleitungseffekte berücksichtigt. Bei derartigen Signalaufbereitungseffekten und/oder Signalweiterleitungseffekten kann es sich beispielsweise um eine Signalberechnung in Computern, Zeitverzögerungen durch Signalweiterleitungen in Computernetzwerken (beispielsweise Ethernet oder sonstige paketorientierte Leistungssysteme) und ähnliches handeln. Diese Zeitverzögerungen sind oftmals relativ konstant, können sich jedoch auch ändern, wenn beispielsweise ein Computerprogramm mit einem adaptiven Algorithmus benutzt wird, und eine größere Anzahl von Iterationsschritten durchgeführt wird. Eine Notwendigkeit hierzu kann sich beispielsweise ergeben, wenn eine höhere Genauigkeit erzielt werden soll und/oder bei gleichbleibender Genauigkeit ein größeres Zielvolumen bestrahlt werden soll. Insbesondere kann es sich bei den Signalen um eine oder mehrere Steuereingaben handeln.

Besonders vorteilhaft ist es, wenn bei dem Verfahren die Steuereingabe der Bestrahlungsvorrichtung zumindest zeitweise und/oder zumindest teilweise dazu genutzt wird, um die Bewegung eines sich zumindest zeitweise und/oder zumindest bereichsweise bewegenden Zielkörpers zu kompensieren. Die Bewegung ist dabei nicht notwendigerweise auf den Zielkörper als ganzem begrenzt. Vielmehr ist es auch denkbar, dass sich der Zielkörper zumindest zum Teil "in sich" bewegt. Im Extremfall ist es auch denkbar, dass sich die äußere Hülle des Zielkörpers (im Wesentlichen) nicht verändert, sich jedoch Teile innerhalb des Zielkörpers gegeneinander verschieben. Im Übrigen ist bei einer "Verschiebung" nicht nur an Translationsfreiheitsgrade, sondern vielmehr auch an Dichteveränderungen, Rotationsfreiheitsgrade und dergleichen zu denken.

Besonders vorteilhaft kann es auch sein, wenn bei dem Verfahren zumindest zeitweise und/oder zumindest bereichsweise ein zeitlicher Vorhersagealgorithmus verwendet wird. Insbesondere ist es möglich, dass basierend auf der aktuellen bzw. der vergangenen Bewegung eines Zielkörpers und/oder eines Zielvolumenbereichs eines Zielkörpers eine Vorhersage über die weitere Bewegung des Zielkörpers/des Zielvolumenbereichs, insbesondere für die nähere Zukunft, gemacht wird. Hierdurch können insbesondere die vorab beschriebenen Verzögerungszeitspannen (gegebenenfalls auch sonstige Zeitverzögerungen) berücksichtigt werden. Weiterhin kann die zeitliche Vorhersage in einem zeitlichen Bereich liegen, der im Wesentlichen mit den Verzögerungszeitspannen übereinstimmt. Die Vorhersage kann sich im Übrigen auch auf die Vorhersage der (voraussichtlichen) Länge eines variierenden Zeitintervalls (beispielsweise auf eine variierende Verzögerungszeitspanne) beziehen. Auf diese Weise kann das Bestrahlungsergebnis nochmals gegebenenfalls deutlich verbessert werden. Der Vorhersagealgorithmus kann beispielsweise durch lineare Extrapolation, kubische Extrapolation, Spline-Extrapolation, neuronale Netze, Markov-Ketten oder sonstige Algorithmen (gegebenenfalls auch analytischer Natur) erfolgen. Um die Genauigkeit des zeitlichen Vorhersagealgorithmus weiter zu erhöhen, ist es auch denkbar, dass die Werte, auf denen die Extrapolation beruht, zumindest in einem gewissen Maß gemittelt werden, um so "statistische Ausreißer" bzw. Rauscheffekte verringern bzw. sogar weitgehend vermeiden zu können.

Besonders bevorzugt ist es, wenn das Verfahren derart durchgeführt wird, dass zumindest zeitweise und/oder zumindest bereichsweise die Steuereingabe derart erfolgt, dass unter Berücksichtigung zumindest einer Verzögerungszeitspanne ein zu bestrahlender Zielvolumenbereich eines Zielkörpers ausgewählt wird. Besonders vorteilhaft ist es, wenn eine Mehrzahl, vorzugsweise (im Wesentlichen) sämtliche relevanten Verzögerungszeitspannen berücksichtigt werden. Dies betrifft insbesondere Verzögerungszeitspannen welche (zumindest teilweise) "sequenziell" und/oder (zumindest teilweise) "parallel" zueinander liegen. Die Steuereingabe kann also derart erfolgen, dass durch diese der Volumenbereich (insbesondere entsprechender Rasterpunkt im Volumenbereich) angesteuert wird, von dem unter Berücksichtigung zumindest einer Verzögerungszeitspanne (gegebenenfalls auch weiterer Eingaben) zu erwarten ist, dass er sich zum Zeitpunkt der tatsächlichen Bestrahlung dort befindet, wo die Strahlung der Bestrahlungsvorrichtung tatsächlich einwirkt. Bei entsprechender Wahl der Steuereingabe hat sich nämlich aufgrund der Bewegung des Zielkörpers beziehungsweise des Zielvolumenbereichs, der Zielkörper bzw. der Zielvolumenbereich zu dem Zeitpunkt, an dem die Strahlung schlussendlich von der Bestrahlungsvorrichtung im Bereich des Zielkörpers bzw. des Zielvolumenbereichs freigesetzt wird, in den entsprechenden, bestrahlten Bereich bewegt. Dadurch wird der "eigentlich gemeinte" Zielvolumenbereich bestrahlt. Mit anderen Worten wird zum Zeitpunkt der Steuereingabe auf einen eigentlich "falschen" Zielvolumenbereich gezielt. Dies erfolgt jedoch so, dass zum Zeitpunkt der eigentlichen Bestrahlung der "richtige" Zielvolumenbereich getroffen wird. Besonders vorteilhaft kann die vorgeschlagene Weiterbildung des Verfahrens sein, wenn es zumindest zeitweise und/oder zumindest bereichsweise mit einem zeitlichen Vorhersagealgorithmus kombiniert wird.

Eine besonders vorteilhafte Weiterbildung des Verfahrens kann sich ergeben, wenn bei der Steuereingabe zumindest zeitweise und/oder zumindest teilweise eine zeitlich variable und/oder sich zeitlich verändernde Strahlungsintensität der Bestrahlungsvorrichtung berücksichtigt wird. Beispielsweise bei Teilchenbeschleunigern tritt häufig der Effekt auf, dass die freigesetzte Dosisleistung (Teilchenfluss) auch während einer Extraktionsphase nicht konstant ist. Eine derartige zeitlich variable und/oder sich zeitlich verändernde Bestrahlungsintensität kann insbesondere bei der Bestrahlung bewegter Zielvolumenbereiche Auswirkungen auf die durchzuführende Strahlnachführung, und damit auf die anzulegenden Steuerparameter haben. Denn wenn beispielsweise eine hohe Strahlungsintensität vorliegt, so ist die pro Rasterpunkt zu deponierende Teilchenanzahl nach einer entsprechend kurzen Zeitdauer deponiert. Bei niedriger Bestrahlungsintensität ist die Bestrahlungszeit pro Rasterpunkt entsprechend länger. Dementsprechend ist beispielsweise der fünfnächste zu bestrahlende Rasterpunkt je nach Bestrahlungsintensität bereits nach 10 ms oder erst nach 30 ms zu bestrahlen. Umgekehrt formuliert: bei der Anwendung von Ortskorrekturen bezüglich der (hauptsächlichen) Bestrahlungseinwirkung muss sich bei Berücksichtigung einer Zeitverzögerung von beispielsweise 20 ms die Ortskorrektur beispielsweise bereits auf den drittnächsten oder aber auch erst auf den 20-nächsten Rasterpunkt beziehen. Die vorliegend genannten Zahlenwerte (Zeit/Rasterpunktnummer) sind selbstverständlich lediglich beispielhaft genannt und können im konkreten Fall auch andere Werte einnehmen. Problematisch ist hierbei oftmals, dass das Extraktionsprofil starken Tagesschwankungen oder Schwankungen von Bestrahlungstag zu Bestrahlungstag unterliegen kann, und dementsprechend nicht oder nicht mit ausreichender Genauigkeit bei der Bestrahlungsplanung berücksichtigt werden kann. Es hat sich jedoch gezeigt, dass das Bestrahlungsprofil (Extraktionsprofil) bei aufeinander folgenden Extraktionszyklen in der Regel relativ ähnlich zueinander ist. Eine signifikante Veränderung des Extraktionsprofils tritt in der Regel auf einer Zeitskala von mehreren Stunden oder länger auf. Dementsprechend kann auch eine Messung, die das Extraktionsprofil (oder eine sonstige zeitlich variable und/oder sich zeitlich verändernde Strahlungsintensität der Bestrahlungsvorrichtung) berücksichtigt, zur Verbesserung des Bestrahlungsverfahrens verwendet werden, wobei Messungen typischerweise im Abstand von mehreren Stunden und/oder einmal oder mehrmals am Tag und/oder unmittelbar vor einer Behandlung durchgeführt werden können. Wird eine derartige zeitlich variierende oder variable und/oder sich zeitlich verändernde Strahlungsintensität der Bestrahlungsvorrichtung berücksichtigt, so kann die in einzelne Untervolumen des Zielvolumenbereichs zu deponierende Strahlungsdosis (beispielsweise die in einem einzelnen Rasterpunkt des Zielvolumenbereichs zu deponierende Strahlungsdosis) besonders genau erfolgen, was vorteilhaft ist. Besonders große Vorteile lassen sich in der Regel in Kombination mit den unterschiedlichen Arten von Scanning-Verfahren erzielen.

Vorteilhaft ist es auch, wenn bei dem Verfahren eine bereits erfolgte Bestrahlung eines Volumenbereichs des Zielkörpers einen Einfluss auf die Steuereingabe einer noch zu erfolgenden Bestrahlung eines Volumenbereichs des Zielkörpers hat. Insbesondere im medizinischen Bereich ist es oftmals ohnehin erforderlich, dass eine Behandlung in Form mehrerer zeitlich zueinander beabstandeter Fraktionen erfolgt (zwischen einzelnen Fraktionen liegen typischerweise Stunden und/oder Tage). Dies kann erforderlich sein, damit das differentielle Reperaturvermögen von Tumor und Normalgewebe eine erhöhte integrale Tumordosis erlaubt. Wenn die aktuelle Bestrahlung jeweils mitprotokolliert wird, so kann der bereits erfolgte Dosiseintrag bei der Bestrahlungsplanung für die nächste Fraktion (bzw. die nächsten Fraktionen) entsprechend berücksichtigt werden. Auf diese Weise kann die Genauigkeit der Bestrahlung nochmals erhöht werden. Zusätzlich oder alternativ ist es jedoch auch möglich, dass eine derartige Anpassung auch innerhalb einer einzelnen Fraktion erfolgt. So wird eine einzelne Fraktion heutzutage oftmals in Form mehrerer Einzeldurchgänge appliziert. In der Regel werden dabei unterschiedliche Einzeldurchgänge aus unterschiedlichen Einstrahlrichtungen appliziert. Dabei kann beispielsweise ein Einzeldurchgang (bzw. können mehrere Einzeldurchgänge) einen Einfluss auf die noch zu applizierenden folgenden Einzeldurchgänge haben. Auch hier kann die Qualität der Bestrahlung nochmals erhöht werden.

Vorteilhaft kann es auch sein, wenn die Bestrahlung zumindest teilweise und/oder zumindest bereichsweise als ein Scanning-Verfahren, insbesondere als ein Raster-Scanning-Verfahren, bevorzugt als ein intensitätsgesteuertes Raster-Scanning-Verfahren durchgeführt wird. Derartige Scanning/Raster-Scanning-Verfahren sind für das vorab beschriebene Verfahren und/oder für dessen Weiterbildungen in der Regel besonders vorteilhaft. Bei Verwendung von Scanning/Raster-Scanning-Verfahren kann beispielsweise auch ein einzelner dünner Strahl (zum Beispiel ein Teilchenstrahl, insbesondere ein so genannter "Bleistiftstrahl" beziehungsweise "Pencilstrahl") zur Beaufschlagung eines relativ großen Zielvolumenbereichs verwendet werden, wobei der Zielvolumenbereich durch den Bleistiftstrahl abgetastet oder abgescannt wird. Dies ist vorteilhaft, da der Strahl nicht aufgeweitet werden muss, was typischerweise zu Ungenauigkeiten bei der Bestrahlung führen würde.

Besonders vorteilhaft kann es sein, wenn bei dem Verfahren die Erzeugung der Steuereingabe zumindest zeitweise und/oder zumindest teilweise unter Verwendung einer Bestrahlungsplanung, insbesondere zumindest zeitweise und/oder zumindest teilweise unter Verwendung einer vorab erstellten und/oder einer im Verlauf der Bestrahlung erstellten und/oder angepassten Bestrahlungsplanung erfolgt. Es hat sich gezeigt, dass eine Bestrahlungsplanung, die typischerweise numerisch relativ komplex ist und oftmals auch als iteratives Verfahren unter Verwendung einer großen Anzahl von Iterationsschritten, erstellt wird, mehrere Minuten und sogar Stunden Berechnungszeit erfordern kann, selbst wenn hierfür sehr schnelle Rechner verwendet werden. Durch eine (teilweise) Entkopplung der Berechnung der Bestrahlungsplanung und der eigentlichen Bestrahlung kann somit die Genauigkeit der Bestrahlung nochmals erhöht werden. Durch eine (teilweise) Erstellung und/oder Anpassung der Bestrahlungsplanung im Verlauf der Bestrahlung können insbesondere Effekte berücksichtigt werden, welche beispielsweise erst im Verlauf der Behandlung auftreten bzw. bekannt werden.

Als typisches Beispiel hierfür ist beispielsweise der Extraktionsverlauf bei einer Teilchenbeschleunigervorrichtung zu nennen und/oder sonstige Maschinenparameter und/oder bestimmter Patientenparameter (beispielsweise die exakte Lage eines Tumorgebiets und/oder eine Bewegung des Patienten).

Besonders vorteilhaft ist es, wenn es sich bei der Bestrahlungsvorrichtung zumindest teilweise um eine Partikelstrahlvorrichtung oder Teilchenstrahlvorrichtung, insbesondere um eine Schwerionen-Beschleunigervorrichtung, um eine Protonen-Beschleunigervorrichtung, um eine Hadronen-Beschleunigervorrichtung, um eine Pionen-Beschleunigervorrichtung, um eine Helimionen-Beschleunigervorrichtung, um eine lineare Beschleunigervorrichtung, um eine Synchotron-Beschleunigervorrichtung, um eine Leptonen-Beschleunigervorrichtung und/oder um eine Elektronen-Beschleunigervorrichtung handelt. Speziell Partikelstrahlen oder Teilchenstrahlen sind bei der Zerstörung von Tumorgewebe, bzw. bei der Beaufschlagung von bestimmten Materialien besonders effektiv. Darüber hinaus kann speziell bei der Verwendung von Partikeln oder Teilchen oder Ionen der Effekt der Energieabgabe im Zielvolumen in Form des sogenannten Bragg-Peak genutzt werden. Wenn Teilchen durch ein Material hindurch treten, verlieren diese ihre Energie nicht in gleichmäßigen "Portionen", insbesondere nicht kontinuierlich. Vielmehr ist die Energieabgabe auf einen Bereich konzentriert, der kurz vor dem Punkt liegt, an dem die Teilchen vollständig im Material deponiert werden, anders ausgedrückt: "steckenbleiben" - der überwiegende Teil der Energieabgabe erfolgt im sogenannten Bragg-Peak. Hierdurch ist eine Variation des Dosiseintrags in Richtung des Teilchenstrahls (in z-Richtung) möglich, indem die Lage des Braggpeaks im Zielvolumen verändert, insbesondere gezielt verändert wird. Dadurch kann eine Bestrahlung auch innerhalb eines Zielkörpers erfolgen, auch ohne dass die um den Zielvolumenbereich des Zielkörpers liegenden Bereiche (in größerem Maße) mit der Strahlung beaufschlagt werden. Hierbei wird die Lage des Bragg-Peaks derart gesteuert, dass dieser nur im Zielvolumenbereich liegt.

Weiterhin wird eine Bestrahlungsvorrichtung zur Bestrahlung zumindest eines Zielvolumenbereichs eines Zielkörpers vorgeschlagen, die zumindest eine Bestrahlungs-Anpassvorrichtung aufweist, wobei die Bestrahlungsvorrichtung zumindest eine insbesondere elektronische Steuervorrichtung aufweist, welche derart ausgebildet und eingerichtet ist, dass sie ein Verfahren mit den vorab beschriebenen Merkmale durchführt. Auch die Bestrahlungsvorrichtung weist die vorab im Zusammenhang mit dem Verfahren beschriebenen Eigenschaften und Vorteile in analoger Weise auf. Bei der elektronischen Steuervorrichtung kann es sich beispielsweise um einen elektronischen Rechner (Computer) handeln oder um ein elektronisches Hardwaremodul. Dieses kann die Steuersignale aus einer Speichervorrichtung abrufen und/oder verarbeiten oder auch online berechnen.

Im Folgenden wird die Erfindung anhand vorteilhafter Ausführungsbeispiele und unter Bezugnahme auf die beigefügte Zeichnung näher erläutert. Es zeigen:
- Fig. 1:: eine Bestrahlungsvorrichtung zur Bestrahlung eines innenliegenden Volumenbereichs eines zu bestrahlenden Körpers in einer schematischen Prinzipdarstellung;
- Fig. 2:: eine Veranschaulichung einer 4-D-Bestrahlungsplanung;
- Fig. 3:: eine schematische Veranschaulichung einer Zielpunkt-Vektorkorrektur;
- Fig. 4:: eine Atemverlaufskurve als Beispiel für ein zyklisches, wiederkehrendes Bewegungsmuster;
- Fig. 5:: ein schematischer Ablaufplan für ein Verfahren zur verzögerungszeitkorrigierten Bestrahlung;
- Fig. 6:: ein schematischer Ablaufplan für ein Verfahren zur Bestimmung von korrigierten Zielkoordinaten.

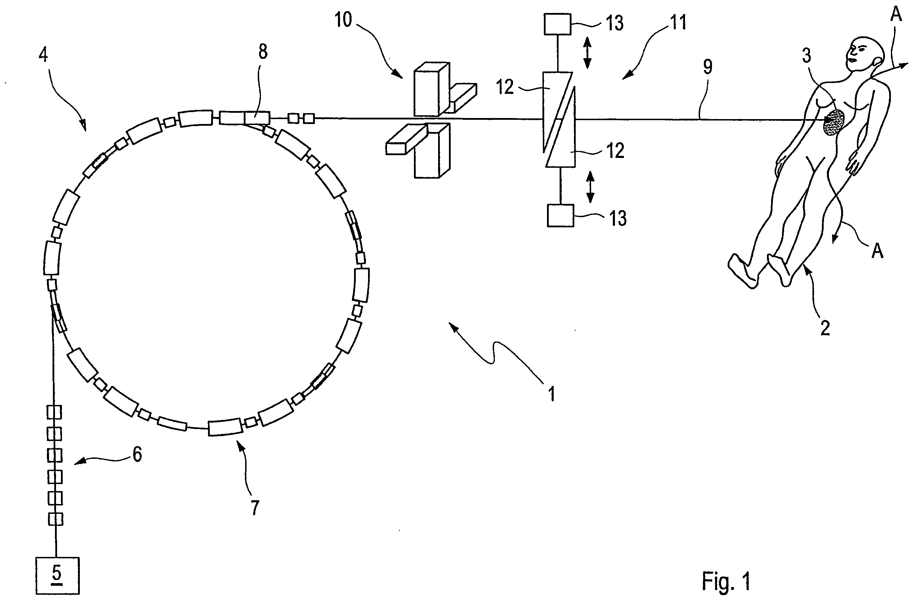

In Fig. 1 ist in einer schematischen Prinzipdarstellung eine Bestrahlungsvorrichtung 1 zur Bestrahlung eines in einem Zielkörper 2 liegenden Zielvolumenbereichs 3 dargestellt. Die Bestrahlungsvorrichtung 1 ist im vorliegend dargestellten Ausführungsbeispiel als Synchotron-Teilchenbeschleuniger 4 mit einem an sich bekannten Aufbau ausgebildet. Der Synchotron-Teilchenbeschleuniger 4 weist eine Ionenquelle 5 auf, in der einzelne Atome erzeugt und ionisiert werden, mithin als Ionen vorliegen. Insbesondere für medizinische Zwecke haben sich Helium-Ionen, Protonen, Neon-Ionen und insbesondere Kohlenstoff-Ionen als besonders vorteilhaft und effektiv erwiesen. Ein großer Vorteil bei den genannten Teilchensorten besteht insbesondere darin, dass diese - speziell Kohlenstoff - biokompatibel sind, mithin bei einer Implantation in ein lebendes organisches Gewebe keine Gewebeunverträglichkeiten erzeugen. Darüber hinaus besteht ein Vorteil der genannten Teilchen (wie auch von sonstigen Schwerionen) darin, dass deren Energiedeposition im Gewebe einen relativ scharf ausgeprägten Bragg-Peak aufweist. Über die Teilchenenergie des entsprechenden Teilchens ist somit die Eindringtiefe des Teilchenstrahls in Materie, insbesondere im Gewebe steuerbar (und damit insbesondere der Bereich der größten Energiefreisetzung steuerbar, um beispielsweise einen Tumor im Inneren eines Körpers zu therapieren). Selbstverständlich ist auch die Verwendung anderer Partikel oder Teilchen denkbar, wie beispielsweise Protonen, Elektronen und Pionen und/oder aber auch ganz allgemein Leptonen und Hadronen (wobei die Bestrahlungsvorrichtung 1 gegebenenfalls zu modifizieren ist).

Die von der Ionenquelle 5 freigesetzten Teilchen werden zunächst in einem Linearbeschleuniger 6 vorbeschleunigt, bevor sie in den eigentlichen Beschleunigerring 7 des Synchotron-Teilchenbeschleunigers 4 eingeschossen werden. Die im Beschleunigerring 7 befindlichen Teilchen werden anschließend auf die gewünschte Energie beschleunigt. Sobald diese Energie erreicht ist, wirkt der Beschleunigerring 7 als Speicherring und die hochenergetischen Teilchen werden über ein Extraktionsseptum 8 langsam aus dem Beschleunigerring 7 entnommen, typischerweise extrahiert (typische Extraktionszeiten liegen im Bereich von einigen Sekunden bis Dekasekunden). Der extrahierte Teilchenstrahl 9 wird mit Hilfe einer Ablenkvorrichtung 10, die vorliegend in Form zweier zueinander senkrecht angeordneter Magnetspulenpaare ausgebildet ist, in einer lateral zur Teilchenstrahlrichtung 9 liegenden Richtung abgelenkt. Auf diese Weise kann im Bereich des Zielkörpers 2 ein beliebiger Punkt in einer x-y-Ebene (steht im Wesentlichen senkrecht zum Teilchenstrahl 9) erreicht werden (wobei die maximale Größe der x-y-Ebene durch die genaue Bauausführung der Bestrahlungsvorrichtung 1 bestimmt wird). Auf diese Weise kann das Zielvolumenbereich 3 im Zielkörper 2 mit einem Scan-Verfahren sukzessive "angefahren" werden. Insbesondere können einzelne Volumeneinheiten oder Volumenbereiche im Zielvolumenbereich 3, im allgemeinen als Rasterpunkte bezeichnet, gezielt und sukzessive angefahren werden.

Wenn die Teilchenenergie des Teilchenstrahls 9 konstant bleibt, werden dabei im Zielvolumenbereich 3 Punkte erreicht, die in einer sogenannten Iso-Energieebene liegen. Die Iso-Energieebene liegt dabei im Wesentlichen senkrecht zur Richtung des Teilchenstrahls 9. Die Tiefenlage der Iso-Energieebene entspricht im Wesentlichen der Lage der Bragg-Peaks. Um das Zielvolumen 3 im Zielkörper 2 auch in z-Richtung (parallel zur Richtung des Teilchenstrahls 9) durchscannen zu können, ist es daher erforderlich die Energie der Teilchen im Teilchenstrahl 9 zu variieren. Heutige Synchrotron-Teilchenbeschleuniger 4 können jedoch die Teilchenenergie bestenfalls zwisehen zwei aufeinanderfolgenden Extraktionszyklen variieren. Für die Behandlung eines Patienten würden sich damit nicht-vertretbar lange Behandlungszeiten ergeben.

Um eine schnelle Variation der Teilchenenergie durchführen zu können, wird im vorliegenden Beispiel ein passiver Energiemodulator 11 verwendet. Der passive Energiemodulator 11 besteht aus zwei im Wesentlichen identisch zueinander ausgebildeten, jedoch symmetrisch zueinander angeordneten Keilen 12 aus einem energieabsorbierenden Material. Die Keile 12 sind beispielsweise unter Verwendung von Stellmotoren 13 (beispielsweise Linearmotoren) in einer im Wesentlichen senkrecht zur Richtung des Teilchenstrahls 9 stehenden Richtung verschiebbar (jeweils durch einen Doppelpfeil angedeutet). Die Bewegungen der beiden Keile 12 sind dabei miteinander synchronisiert. Je nach Stellung der beiden Keile 12 muss der Teilchenstrahl 9 eine unterschiedliche Länge durch das energieabsorbierende Material der beiden Keile 12 hindurch laufen. Dementsprechend wird die Energie der Teilchen des Teilchenstrahls 9 entsprechend stark vermindert. Auf diese Weise ist die Energie der Teilchen, die am eigentlichen Zielkörper 2 ankommen, variabel, sodass sich der Bragg-Peak verschiebt und somit das Zielvolumen 3 auch in z-Richtung gescannt werden kann.

Als Scanverfahren wird heutzutage oftmals ein sogenanntes Raster-Scanverfahren verwendet, bei dem das zu bestrahlende Zielvolumen oder der Zielvolumenbereich 3 (üblicherweise auch ein Teil des das Zielvolumen 3 umgebenden Gewebes des Zielkörpers 2) in eine größere Anzahl kleiner Volumeneinheiten, sogenannte Rasterpunkte, aufgeteilt wird. Der Teilchenstrahl 9 fährt dabei nacheinander jeden einzelnen Rasterpunkt an. Unter Berücksichtigung der extrahierten Intensität wird über entsprechende Detektoren und die Verweildauer des Teilchenstrahls 9 an den einzelnen Rasterpunkten sichergestellt, dass die in der Bestrahlungsplanung optimierten Dosis im Rasterpunkt deponiert wird. Die Implikationen, die solche Intensitätsschwankungen mit sich bringen, werden im Folgenden noch näher erläutert.

Die Intensitätsschwankungen rühren vom so genannten "Extraktionsspektrum" her. Auch wenn versucht wird, die Beschleunigervorrichtungen (wie beispielsweise die Bestrahlungsvorrichtung 1) derart auszuführen, dass während der Extraktionsphase ein möglichst konstanter, homogener Teilchenfluss aus dem Beschleunigerring 7 entnommen wird, so sind zumindest gewisse Schwankungen des Teilchenflusses in aller Regel nicht vermeidbar. Es hat sich jedoch gezeigt, dass sich das Extraktionsspektrum - obgleich an sich nicht konstant - während des Betriebs der Bestrahlungsvorrichtung 1 nur relativ langsam ändert. Typischerweise erfolgt eine signifikante Veränderung über eine Zeitskala von mehreren Stunden hinweg. Von daher ist es möglich, dass das Extraktionsspektrum beispielsweise am Anfang eines Behandlungstages, beziehungsweise am Anfang einer einzelnen Behandlung gemessen wird, und das so gemessene Extraktionsspektrum als Grundlage für die weiteren Berechnungen herangezogen wird. Es ist auch möglich, dass eine geplante Behandlung (siehe folgende Beschreibung) vorab in eine leere Kavität beziehungsweise in ein Phantom oder einen Dummy eingebracht wird. Hierdurch ist es insbesondere möglich, den späteren, zeitlichen Ablauf der Behandlung zumindest näherungsweise vorab zu ermitteln.

Bei heutigen Behandlungsverfahren wird typischerweise vorab eine sogenannte Bestrahlungsplanung erstellt. Hierbei wird - grob gesprochen - auf Basis von Bildgebungsdaten, physikalischen Basisdaten, (biologischen) Modellen und der von einem Arzt verschriebenen (biologisch wirksamen) Dosisverteilung ein Steuerdatensatz für die Bestrahlungsvorrichtung 1 optimiert.

In der Praxis erfolgt die Bestrahlungsplanung dadurch, dass berechnet wird, welchen biologischen Effekt ein Teilchenstrahl 9, der aus einer oder aus mehreren Richtungen mit einem bestimmten (dreidimensionalen) Bewegungsmuster (Scanning-Verfahren) in den Zielvolumenbereich 3 des Zielkörpers 2 eingebracht wird, verursacht. Die so berechneten biologischen Effekte werden mit der vom Arzt verschriebenen biologisch wirksamen Dosisverteilung verglichen. Durch Optimierungsverfahren wird versucht, die Differenz zwischen der verschriebenen Dosisverteilung und den berechneten biologischen Effekten zu minimieren. Es hat sich dabei bewährt, den Teilchenstrahl 9 aus zwei unterschiedlichen (insbesondere einander entgegengesetzten) Richtungen zeitlich nacheinander auf den Zielvolumenbereich 3 des Zielkörpers 2 zu richten.

Im Rahmen einer Bestrahlungsplanung ist eine Vielzahl von unterschiedlichen Effekten zu berücksichtigen. So werden beispielsweise auch Dosisbeiträge berücksichtigt, die der Teilchenstrahl in anderen Rasterpunkten einbringt. Dabei sind die Dosiseinträge in aller Regel hinter dem "aktuellen" Rasterpunkt sehr klein (sodass diese oftmals vernachlässigt werden können), wohingegen sie in Strahlrichtung gesehen vor dem "aktuellen" Rasterpunkt durchaus relevante Dosiseinträge bewirken können. Weiterhin ist - insbesondere im Falle von Schwerionen-Teilchenstrahlung - zu berücksichtigen, dass die so genannte relative biologische Wirksamkeit (RBE für Englisch: "relative biological effectiveness") in komplexer und nichtlinearer Weise von physikalischen Parametern abhängen. Beispielsweise kann sich der Zusammenhang zwischen der deponierten physikalischen Dosis (korrespondierend mit dem Energieverlust des Teilchenstrahls) und der Gewebeschädigung (also der biologisch wirksamen Dosis) in Abhängigkeit von der Teilchenenergie verändern. Auch kann es - wiederum insbesondere im Falle von Schwerionen-Teilchenstrahlung - zu so genannter Sekundärstrahlung durch Fraktionierung,vom Schwerionenstrahl induzierte Teilchen kommen. Auch dies bringt nichtlineare biologische Effekte mit sich. Weiterhin verändert sich die eingebrachte Dosis (sowohl die physikalische, als auch die biologisch wirksame Dosis) mit der Gewebeart. Mithin sind (unter anderem) Knochen, Muskelgewebe, Blutgefäße, Hohlräume und dergleichen bei der Bestrahlungsplanung unterschiedlich zu gewichten. Bei einer großen Anzahl an Rasterpunkten (in der Praxis können ohne weiteres 20.000-50.000 Rasterpunkte zu berücksichtigen sein) kann die Erstellung einer Bestrahlungsplanung erhebliche Zeitdauern (durchaus mehrere Stunden) beanspruchen, auch wenn sehr schnelle Computer verwendet werden. Ergänzende Informationen zur Erstellung von Bestrahlungsplanungen finden sich insbesondere in den beiden Artikeln "Treatment planning for heavy ion radiotherapy: clinical implementation and application" von M. Krämer, O. Jäckel, T. Haberer, G. Kraft, D. Schardt und U. Weber in Phys. Med. Biol., Vol. 45, Jahrg. 2000, Seite 3299-3317, sowie "Treatment planning for heavy ion radiotherapy: calculation and optimization of biologically effective dose" von M. Krämer und M. Scholz in Phys. Med. Biol., Vol. 45, Jahrg. 2000, Seite 3319-3330.

Ein zusätzliches Problem ergibt sich, wenn sich der Zielkörper 2 und/oder der im Zielkörper 2 befindliche Zielvolumenbereich 3 bewegt. Dies ist in Fig. 1 durch Pfeile A angedeutet. Die Formgebung der Pfeile A soll dabei andeuten, dass die Bewegung des Zielvolumenbereichs 3 nicht unbedingt auf eine lineare Bewegung beschränkt ist. Vielmehr kann die Bewegung entlang einer unregelmäßig geformten Kurve erfolgen. Dabei kann es nicht nur zu Bewegungen in Form von Translationsfreiheitsgraden kommen, sondern darüber hinaus kann es noch zu Rotationsbewegungen, sowie zu Dichteänderungen in den beteiligten Geweben kommen (insbesondere Letzteres kann zu einer stärken und/oder schwächeren Absorption des Teilchenstrahls 9 führen, sodass sich die Lage des Bragg-Peak - und damit die Iso-Energieebene - verschiebt). Eine derartige Bewegung des Zielvolumenbereichs 3 kann sich beispielsweise dann ergeben, wenn ein Tumor behandelt werden soll, der sich in oder in der Nähe der Lunge befindet (Atembewegung), sich in oder in der Nähe des Herzens befindet (Herzschlag) oder sich im Bereich des Darms befindet (Darmperistaltik).

Um bei derartig bewegten Zielvolumenbereichen 3 eine gezielte Bestrahlung unter Verwendung einer Bestrahlungsplanung durchführen zu können, wird vorab die Bewegung des Zielvolumenbereichs 3, insbesondere der Verlauf der dessen Bewegung untersucht. Beispielsweise können hierfür bildgebende Verfahren, wie beispielsweise ein sogenanntes 4-D-CT-Verfahren (CT für Computertomographie) verwendet werden. Für die Überwachung der eigentlichen Therapie sind CT-Verfahren eher unerwünscht, da diese einerseits eine zusätzliche (unerwünschte) Strahlenbelastung mit sich bringen, und darüber hinaus relativ langsam sind. Daher wird bei der Messung der Zielvolumenbereichsbewegung 3 typischerweise gleichzeitig ein Ersatzsignal aufgenommen. Hierbei kann es sich beispielsweise um eine Videoaufnahme der entsprechenden Körperregion, um die Bewegung implantierter Sensoren oder um Dehnungsmessstreifen oder dergleichen handeln. Wurde die Korrelation zwischen Ersatzsignal und der eigentlichen Bewegung des Zielvolumenbereichs 3 ermittelt, so kann die Lage des Zielvolumenbereichs 3 mit recht guter Genauigkeit auch alleine anhand des Ersatzbewegungssignals ermittelt werden.

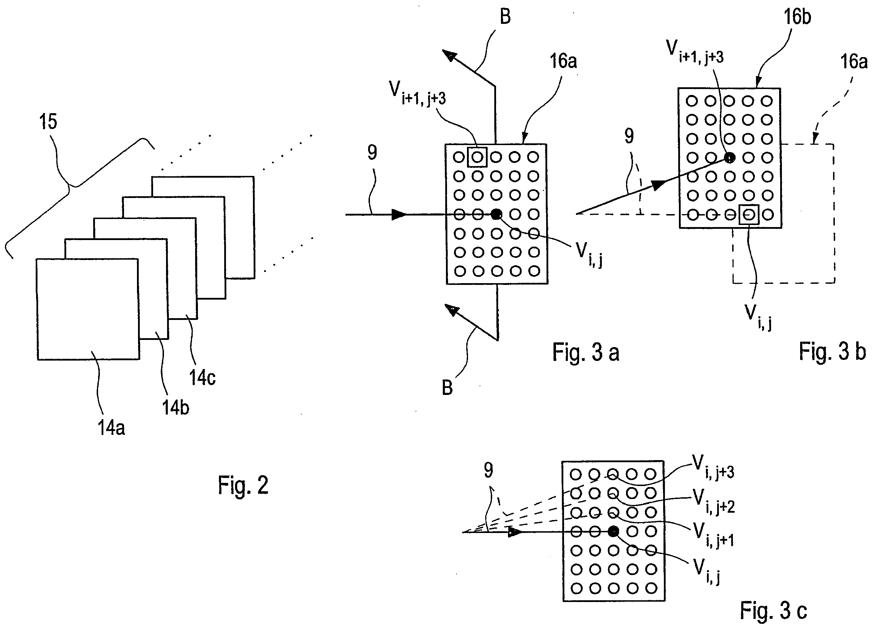

Die Bestrahlungsplanung erfolgt nun dadurch, dass anstelle eines einzelnen Planungsdatensatzes 14 (siehe Fig. 2), eine Vielzahl von unterschiedlichen Planungsdatensätzen 14a, 14b, 14c ... verwendet wird. Jeder Planungsdatensatz 14a, 14b, 14c ... steht dabei für eine bestimmte Bewegungsphase des Zielvolumenbereichs 3. Eine typische Anzahl von Planungsdatensätzen 14a, 14b, 14c ... beträgt zehn Planungsdatensätze 14a, 14b, 14c ...
Bei der eigentlichen Bestrahlung des Zielkörpers 2 wird dann, basierend auf dem Ersatzbewegungssignal (oder einem anderen Signal, welches die Lage des Zielvolumenbereichs 3 beschreibt) der entsprechende, geeignete Planungsdatensatz 14a, 14b, 14c ... verwendet. Hat sich das Zielvolumenbereich 3 ausreichend weit bewegt, so wird auf den nächsten, geeigneten Planungsdatensatz 14a, 14b, 14c ... gewechselt.

Die Erfinder haben nun erkannt, dass dieses Verfahren problematisch sein kann, insbesondere dann, wenn sich das Zielvolumenbereichs 3 relativ schnell bewegt, die Verstellung einzelner Geräteparameter der Bestrahlungsvorrichtung 1 relativ langsam erfolgt und/oder der Teilchenfluss während der Extraktionsphase stark schwankt. Um hier eine für medizinische Zwecke ausreichend große Behandlungsgenauigkeit zu erreichen, ist die Anwendung eines Korrekturvektors erforderlich.

Der Korrekturvektor, der die "Ausgabe" der Bestrahlungsplanung 14a, 14b, 14c ... auf die Steuereingabe der Bestrahlungsvorrichtung 1 transformiert, hängt von mehreren Parametern ab.

Ein erster Parameter ist dabei die Geschwindigkeit der Bewegung des Zielvolumenbereichs 3 beziehungsweise eines Materialausschnitts 16 gegenüber den äußeren Raumkoordinaten. Dies ist in Fig. 3 (insbesondere in Fig. 3a und Fig. 3b) veranschaulicht. Fig. 3 zeigt einen Materialausschnitt 16, wobei in Fig. 3a und Fig. 3b unterschiedliche zeitliche Momentaufnahmen dargestellt sind. Der Zielvolumenbereich 3 (und damit dessen Materialausschnitt 16a) befindet sich in einer in Fig. 3a durch Pfeile B angedeuteten Bewegung. Nach einer gewissen Zeitdauer ist daher der Materialausschnitt 16b (siehe Fig. 3b) gegenüber der ursprünglichen Lage 16a verschoben. In der Realität handelt es sich natürlich um eine dreidimensionale Anordnung von Rasterpunkten. In Fig. 3 ist aus Veranschaulichungsgründen ein lediglich zweidimensionales Zielgebiet dargestellt.

Soll beispielsweise zum Zeitpunkt t₀ (entspricht Fig. 3a) ein Rasterpunkt V_{i,j} bestrahlt werden, so wird das entsprechende "Blatt" 14a, 14b, 14c ... des Gesamtbestrahlungsplans 15 ausgewählt. Dieses Blatt stellt eine Art vorausberechneter "look up"-Tabelle dar, welche die Steuerparameter enthält, mit denen die Bestrahlungsvorrichtung 1 (oder Teile hiervon) angesteuert wird. Hierbei sind insbesondere die Ablenkvorrichtung 10, sowie der Energiemodulator 11 zu nennen.

Bis die einzelnen Komponenten der Bestrahlungsvorrichtung 1 die Sollvorgabe entsprechend den Steuersignalen realisiert haben, hat sich jedoch der Zielvolumenbereich 3 weiter bewegt. Wenn also zu einem späteren Zeitpunkt t₁ (siehe Fig. 3b) der Rasterpunkt V_{i,j} tatsächlich bestrahlt werden soll, so ist der "eigentlich zu bestrahlende Rasterpunkt" bereits weiter gewandert. Fälschlicherweise würde nunmehr der Rasterpunkt V_{i-1,j-3} bestrahlt. Soll zum Zeitpunkt t₁ (Fig. 3b) der "richtige Rasterpunkt" bestrahlt werden, so muss der Teilchenstrahl nunmehr den Rasterpunkt V_{i+1,j+3} bestrahlen. Dies bedeutet wiederum, dass bereits zum Zeitpunkt t₀ Vi_{+1,j+3} als Zielrasterpunkt gewählt werden muss (obwohl dieser zum Zeitpunkt t₀ der "falsche" Rasterpunkt ist), damit zum Zeitpunkt t₁ (an dem die Steuereingabe umgesetzt ist) der "richtige" Rasterpunkt bestrahlt wird.

Einen zweiten Parameter stellt die Zeitverzögerung dar, die zwischen Steuereingabe und Umsetzung der Steuereingabe durch die Beschleunigungsvorrichtung 1 liegt. Dabei kann die Zeitverzögerung durchaus in unterschiedlichen Richtungen unterschiedlich groß ausfallen. So kann die Bewegung B des Materialausschnitts 16 in Fig. 3 in eine Bewegungskomponente longitudinal zum Teilchenstrahl 9, sowie in eine Bewegungskomponente lateral zum Teilchenstrahl 9 aufgeteilt werden. Die Bewegungskomponente lateral zum Teilchenstrahl 9 kann durch eine entsprechende geeignete Ansteuerung der Ablenkvorrichtung 10 ausgeglichen oder kompensiert werden. Hierbei kann eine Steuereingabe relativ schnell umgesetzt werden. Die Verzögerung, die durch die Ablenkvorrichtung 10 induziert wird, liegt typischerweise im Bereich von 1 ms.

Die Bewegungskomponente longitudinal zum Teilchenstrahl 9 muss dagegen durch den Energiemodulator 11 kompensiert werden. Da hier relativ große Massen (die Keile 12) bewegt werden müssen, nimmt hier die zeitliche Verzögerung zwischen Anlegen und Umsetzung einer Steuereingabe eine größere Zeitdauer in Anspruch. Typische Werte liegen im Bereich von 25 ms. Darüber hinaus kommt es noch zusätzlich zu den bereits beschriebenen Zeitverzögerungen zu gewissen Zeitverzögerungen durch die Berechnung der jeweils zu verwendenden Maschinenparameter (beziehungsweise der Steuersignale). Diese sind insbesondere durch die Hardware (Computer, Netzwerktopologie usw.) der Bestrahlungsvorrichtung 1 bestimmt. Typische Werte liegen im Bereich von 5 ms. Es ist im Übrigen darauf hinzuweisen, dass diese Zeitverzögerungen zwar üblicherweise im Laufe einer Bestrahlung relativ konstant bleiben, sich jedoch zwischen zwei unterschiedlichen Bestrahlungen durchaus ändern können (z. Bsp. weil ein größerer Zielvolumenbereich 3 bestrahlt werden muss, sodass eine größere Anzahl an Rasterpunkten verwendet wird, was zu einer entsprechend aufwändigeren Berechnung führen kann.

Einen dritten Parameter für die Richtung und Größe des Korrekturrektors stellt darüber hinaus auch der aktuelle Teilchenfluss (Bestrahlungsintensität) dar, der von der Bestrahlungsvorrichtung 1 bereitgestellt wird. Zur Veranschaulichung soll angenommen werden, dass die in Fig. 3a und Fig. 3b dargestellte, vorab beschriebene Korrektur bei einem bestimmten Teilchenfluss Φ₁ erfolgt. Bei diesem Teilchenfluss Φ₁ erfolgt der Scan-Fortschritt mit einer gewissen Geschwindigkeit, was in Fig. 3c veranschaulicht ist (in Fig. 3c erfolgt aus Veranschaulichungsgründen keine Bewegung des Materialausschnitts 16).

Wenn beispielsweise zum Zeitpunkt t₀ beim Rasterpunkt V_{i,j} gestartet wird, so wird zum Zeitpunkt t₁ der Rasterpunkt V_{i,j+3} bestrahlt. Wie bereits erwähnt ist der Teilchenfluss jedoch nicht konstant, sondern variiert im Lauf eines Extraktionszyklusses. Ist der Teilchenfluss beispielsweise größer als Φ₁, so ist die in den jeweiligen Rasterpunkt einzubringende Teilchenanzahl schneller erreicht und der Scan-Fortschritt entsprechend schneller. Dies hat zur Folge, dass die Bestrahlung des Rasterpunkts V_{i,j+3} bereits zu einem Zeitpunkt t₁' erfolgt, der vor dem Zeitpunkt t₁ liegt. Bei einem niedrigen Teilchenfluss erfolgt der Scan-Fortschritt entsprechend langsamer. Auch dieser Effekt erfordert eine Korrektur in Form eines entsprechend angepassten Korrekturvektors.

Wie insbesondere aus Fig. 3a und Fig. 3b ersichtlich, muss bereits zum Zeitpunkt t₀ die zukünftige Lage des Materialausschnitts 16b zum in der Zukunft liegenden Zeitpunkt t₁ bekannt sein, damit ein geeigneter Korrekturvektor bestimmt werden kann, und die geeigneten Steuersignale erzeugt werden können. Es ist daher erforderlich, dass eine Zeitspanne Δt = t₁ - t₀ "in die Zukunft gesehen" werden muss, wobei die Zeitspanne Δt in unterschiedlichen Richtungen unterschiedlich groß sein kann (wie bereits erläutert wurde) und gegebenenfalls auch vom (sich verändernden) Teilchenfluss abhängen kann.

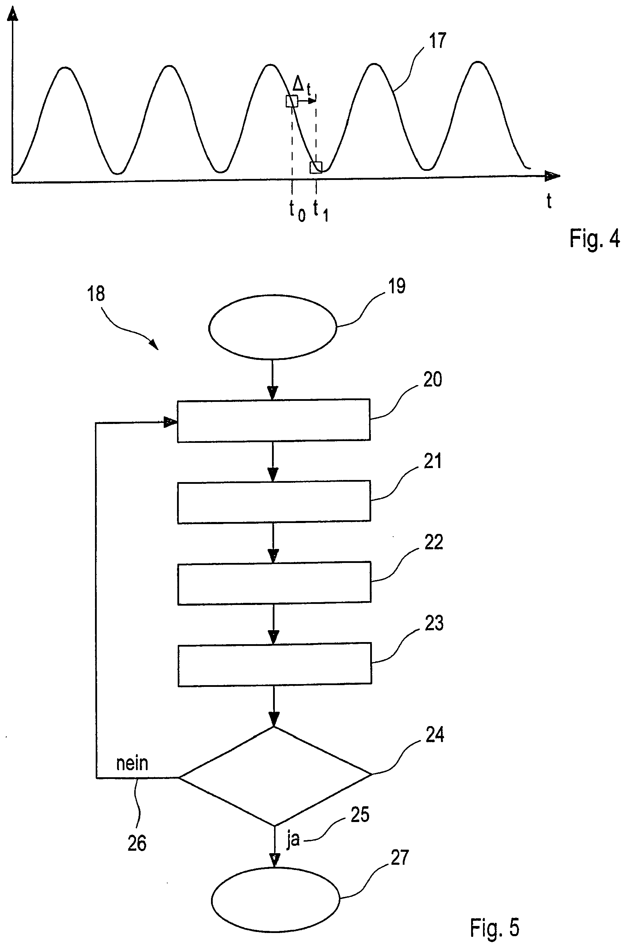

Um derart "in die Zukunft schauen" zu können, ist es erforderlich, den aktuellen Bewegungszustand zum Zeitpunkt t₀ zu extrapolierten (siehe Fig. 4), sodass die Lage des Zielvolumenbereichs 3 zum Zeitpunkt t₁ (zumindest näherungsweise) bekannt ist. Zur Extrapolation können unterschiedlichste Algorithmen verwendet werden. Als vielleicht einfachste Möglichkeit bietet sich dabei die lineare Extrapolation an. Deutlich bessere Ergebnisse können jedoch erzielt werden, wenn beispielsweise bei einem zyklisch wiederkehrenden Bewegungsmuster ein vollständiger, typischer Bewegungszyklus ermittelt, und zur Extrapolation genutzt wird. Dies ist insbesondere deshalb leicht möglich, weil das Bewegungsmuster des Zielvolumenbereichs 3 ohnehin zur Erstellung der Gesamtbestrahlungsplanung 15 (siehe Fig. 2) vorab ermittelt werden muss.

In Fig. 4 ist exemplarisch eine Atembewegungskurve 17 dargestellt. In der Realität wird es zwar üblicherweise zu leichten Abweichungen zwischen der typischen "durchschnittlichen" Atembewegungskurve und der tatsächlichen Atembewegungskurve kommen. Dennoch ist der hierdurch ermittelte Nährungswert typischerweise für praktische Zwecke ausreichend genau. Für den Fall, dass die tatsächliche Atembewegungskurve vom typischen Wert sehr stark abweicht (beispielsweise weil der Patient hustet), so ist es im Übrigen möglich, eine (kurzzeitige) Schnellabschaltung des Teilchenstrahls 9 vorzunehmen, um eine (zu starke) Fehlbestrahlung des Zielkörpers 2 zu vermeiden.

Die Bestimmung des anzusteuernden Zielpunkt, um den Zielvolumenbereich 3 im Zielkörpers 2 zu bestrahlen, erfolgt dann durch eine Faltung der zum eigentlichen Bestrahlungszeitpunkt zu erwartenden Bestrahlungsplanung 14a, 14b, 14c ... und des oben beschriebenen Korrekturvektors. Da die Berechnung des Korrekturvektors üblicherweise zu komplex ist, um online durchgeführt werden zu können, wird gemäß einem bevorzugten Ausführungsbeispiel vorgeschlagen, für sämtliche realistischerweise möglichen Parameterkombinationen der Bestrahlungsvorrichtung 1 jeweils einen zu applizierenden Korrekturwert zu berechnen und in einer Korrekturmatrix geeigneter Größe und Dimension zu speichern. Während der Bestrahlung kann der Korrekturvektor dann aus der Matrix ausgelesen werden.

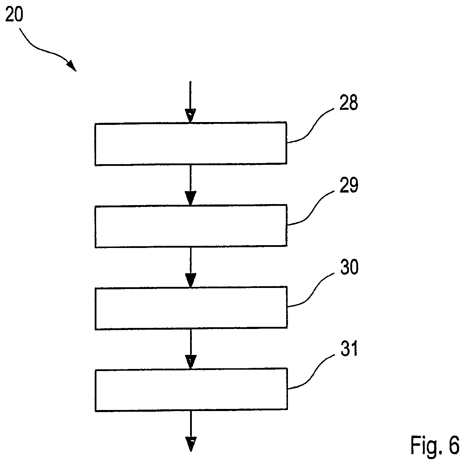

In Fig. 5 ist das Bestrahlungsverfahren 18 nochmals in Form eines schematischen Ablaufdiagramms dargestellt. Am Anfang 19 des Bestrahlungsverfahrens 18 wird die Bestrahlungsvorrichtung 1 initialisiert und die Gesamtbestrahlungsplanung 15 in den Steuerungsrechner (in Fig. 1 nicht dargestellt) der Bestrahlungsvorrichtung 1 eingelesen. Dies kann insbesondere die Ermittlung des aktuellen Extraktionsprofils der Beschleunigervorrichtung 1 beinhalten. In einem ersten Schritt 20 des Bestrahlungsverfahrens 18 wird der jeweils nächste zu bestrahlende Punkt (bzw. am Anfang des Verfahrens der erste zu bestrahlende Punkt) unter Verwendung des jeweiligen Planungsdatensatzes 14a, 14b, 14c ... der Gesamtbestrahlungsplanung 15 sowie des anzuwendenden Korrekturvektors ermittelt. (Die Ermittlung 20 des neuen zu bestrahlenden Punkts ist in Fig. 6 näher dargestellt.) Basierend auf dem derart ermittelten, zu bestrahlenden Punkt wird ein Steuersignal erzeugt 21, welches geeignete Werte zur Ansteuerung der Bestrahlungsvorrichtung 1 aufweist. Beispielsweise handelt es sich hier um die Sollstromstärken für die Ablenkvorrichtung 10 sowie die Sollpositionen für den Energiemodulator 11. Mit den derart ermittelten 21 Steuersignalen werden dementsprechend die einzelnen Maschinenkomponenten angesteuert 22. Mit der entsprechend eingerichteten Bestrahlungsvorrichtung 1 erfolgt somit die Bestrahlung 23 des entsprechenden Rasterpunkts. Ist im entsprechenden Rasterpunkt die gewünschte Dosis deponiert (nach Ablauf der entsprechenden Zeitspanne), so wird im Schritt 24 überprüft, ob die Bestrahlungsplanung bereits vollständig umgesetzt wurde (Zweig 25), oder ob noch weitere Punkte zu bestrahlen sind (Zweig 26). Wird im Überprüfungsschritt 26 festgestellt, dass die Bestrahlung bereits vollständig durchgeführt wurde 25, so wird das Bestrahlungsverfahren 18 im Verfahrensschritt 27 beendet. Wird dagegen festgestellt, dass das Bestrahlungsverfahren 18 noch nicht vollständig beendet ist (Zweig 26), so wird zum Anfang des Verfahrens zurückgesprungen und im Schritt 20 ein neuer zu bestrahlender Punkt ermittelt.

In Fig. 6 ist detaillierter ausgeführt, wie im Schritt 20 ein neuer, zu bestrahlender Punkt ermittelt wird. Dabei wird zunächst aus der Bestrahlungsplanung 15 der zum aktuellen Zeitpunkt zu bestrahlende Punkt ermittelt (Schritt 28).

Im anschließenden Schritt 29 wird (gegebenenfalls auch vorab oder parallel zum Schritt 28) die aktuelle Bewegungsphase des Zielvolumenbereichs 3 erfasst und ermittelt. Hierauf basierend wird eine Vorhersage über die zu erwartende Bewegung des Zielvolumenbereichs 3 gemacht. Dadurch ist es möglich, die zu erwartende Position zu einem späteren Zeitpunkt zu ermitteln, insbesondere zu einem Zeitpunkt, der um die zu erwartende Zeitverzögerung zwischen Anlegen eines Steuersignals und Umsetzung des Steuersignals in der Zukunft liegt, zu bestimmen.

Gleichzeitig wird im Schritt 29 der aktuelle Teilchenfluss (die aktuelle Bestrahlungsintensität) ermittelt. Basierend auf dem aktuellen sowie den vergangenen Werten des Teilchenflusses wird auf die Lage im Extraktionsspektrum geschlossen, so dass die in (näherer) Zukunft liegenden Teilchenflüsse zumindest abgeschätzt werden können. Darauf basierend wird die Geschwindigkeit des Scan-Fortschritts ermittelt.
Unter Berücksichtigung beider Einflüsse werden die anzuwendenden Korrekturwerte (Korrekturvektoren) ermittelt (Schritt 30) und auf die aus der Bestrahlungsplanung 15 ausgelesenen Zielwerte angewendet.
Hierdurch erhält man im Schritt 31 die tatsächlich anzuwendenden, korrigierten Bestrahlungskoordinaten. Diese Koordinaten werden nun für das Bestrahlungsverfahren 18 (Fig. 5) verwendet. Die Erfindung ist in den Ansprüche definiert.

Die Ausführungsbeispiele, die nicht unter den Schutzumfang der beigefügten Ansprüche fallen, dienen lediglich zu illustrativen Zwecken und sind nicht Gegenstand der gegenwärtigen Erfindung. Dies betrifft insbesondere Verweise auf Verfahren, die sich in der Beschreibung auf lebende Körper beziehen, die unter die Ausnahmen der Patentierbarkeit aufgrund chirurgischer oder therapeutischer Verfahren fallen.

Die Erfindung wird durch die folgenden Ansprüche definiert.

**Bezugszeichenliste**

| | | | |
|---|---|---|---|
| 1. | Bestrahlungsvorrichtung | 19. | Anfang |
| 2. | Zielkörper | 20. | Ermittlung Bestrahlungspunkt |
| 3. | Zielvolumenbereich | | |
| 4. | Synchotron-Teilchenbeschleuniger | 21. | Ermittlung Steuersignal |
| | | 22. | Ansteuerung Maschinenkomponenten |
| 5. | Ionenquelle | | |
| 6. | Linearbeschleuniger | 23. | Bestrahlung Rasterpunkt |
| 7. | Beschleunigerring | 24. | Überprüfungsschritt |
| 8. | Extraktionsseptum | 25. | Bestrahlung beendet |
| 9. | Teilchenstrahl | 26. | Bestrahlung noch nicht beendet |
| 10. | Ablenkvorrichtung | | |
| 11. | Energiemodulator | 27. | Ende |
| 12. | Keil | 28. | Ermittlung Zielpunkt |
| 13. | Stellmotor | 29. | Ermittlung Bewegungsphase |
| 14. | Planungsdatensatz | | |
| 15. | Gesamtbestrahlungsplanung | 30. | Ermittlung und Anwendung Korrekturvektor |
| 16. | Materialausschnitt | 31. | Korrigierte Bestrahlungskoordinate |
| 17. | Atembewegungskurve | | |
| 18. | Bestrahlungsverfahren | | |

## Patentansprüche

1. Verfahren (18, 20) zum Betreiben einer Bestrahlungsvorrichtung (1) zur Bestrahlung zumindest eines Zielvolumenbereichs (3) eines nicht-lebenden Zielkörpers (2) mit einem Hadronenstrahl, wobei der zu bestrahlende Zielvolumenbereich (3) des Zielkörpers (2) durch eine Steuereingabe (21) eines Steuerungsrechners der Bestrahlungsvorrichtung (1) verändert werden kann, wobei bei der Erzeugung (20, 21) der Steuereingabe (21)
- eine bereits erfolgte vorherige Bestrahlung eines Zielvolumenbereiches berücksichtigt wird und
- mittels des Steuerungsrechners ein Korrekturvektor angewendet wird wobei der Korrekturvektor von den folgenden Parametern abhängt:
- aktueller Teilchenfluss, und
- Geschwindigkeit der Bewegung des Zielvolumenbereichs (3) oder eines Materialausschnittes (16) gegenüber den äußeren Raumkoordinaten, und
- zumindest einer zwischen der Steuereingabe und einer Reaktion der Bestrahlungsvorrichtung (1) liegende Verzögerungszeitspanne (Δt), die zumindest zeitweise und/oder zumindest bereichsweise berücksichtigt (30) wird, und wobei die Anwendung des Korrekturvektors auf die Steuereingabe und die Steuerung des Hadronenstrahls mittels der Steuereingabe sich so auswirkt, dass der Zielvolumenbereich von dem Hadronenstrahl angesteuert wird, von dem zu erwarten ist, dass er sich zum Zeitpunkt der tatsächlichen Bestrahlung dort befindet, wo die Strahlung der Bestrahlungsvorrichtung tatsächlich einwirkt.

2. Verfahren (18, 20) nach Anspruch 1, **dadurch gekennzeichnet, dass** eine Mehrzahl von Verzögerungszeitspannen (Δt) mit zumindest zeitweise und/oder zumindest teilweise unterschiedlicher und/oder variierender Länge berücksichtigt wird.

3. Verfahren (18, 20) nach Anspruch 1 oder 2, wobei bei der Erzeugung der Steuereingabe berücksichtigt wird, dass bei der Ansteuerung des Zielvolumenbereichs von dem Hadronenstrahl die zwischen der Steuereingabe und der Reaktion der Bestrahlungsvorrichtung (1) liegende Verzögerungszeitspanne (Δt) bei einer lateralen Ablenkung (x-y) des Hadronenstrahls im Vergleich zu einer longitudinalen Ablenkung (z) unterschiedlich groß ausfällt.

4. Verfahren (18, 20) nach einem der vorangehenden Ansprüche, **dadurch gekennzeichnet,**
**dass** die Verzögerungszeitspanne (Δt) bei einer lateralen Ablenkung (x-y) des Hadronenstrahls in einem Bereich unter 250 ms, bevorzugt in einem Bereich unter 100 ms, liegt, und/oder
**dass** die Verzögerungszeitspanne (Δt) bei einer longitudinalen Ablenkung (z) des Hadronenstrahls in einem Bereich unter 150 ms, bevorzugt in einem Bereich unter 80 ms, liegt.

5. Verfahren (18, 20) nach einem der vorangehenden Ansprüche, **dadurch gekennzeichnet, dass** zumindest eine Verzögerungszeitspanne (Δt) Signalaufbereitungseffekte und/oder Signalweiterleitungseffekte berücksichtigt.

6. Verfahren (18, 20) nach einem der vorangehenden Ansprüche, **dadurch gekennzeichnet, dass** zumindest zeitweise und/oder zumindest bereichsweise ein zeitlicher Vorhersagealgorithmus (17) verwendet wird, wobei mittels des zeitlichen Vorhersagealgorithmus basierend auf der aktuellen Bewegung des Zielkörpers und/oder des Zielvolumenbereichs eine Vorhersage über die weitere Bewegung des Zielkörpers und/oder des Zielvolumenbereichs gemacht wird,
wobei der zeitliche Vorhersagealgorithmus durch lineare Extrapolation, kubische Extrapolation, Spline-Extrapolation, neuronale Netze oder Markov-Ketten erfolgen kann.

7. Verfahren (18, 20) nach einem der vorangehenden Ansprüche, **dadurch gekennzeichnet, dass** die Bestrahlung zumindest teilweise und/oder zumindest bereichsweise als ein Scanning-Verfahren, oder als ein Raster-Scanning-Verfahren (18), oder aber als ein intensitätsgesteuertes Raster-Scanning-Verfahren (18) durchgeführt wird.

8. Verfahren (18, 20) nach einem der vorangehenden Ansprüche, **dadurch gekennzeichnet, dass** die Erzeugung der Steuereingabe (21) zumindest zeitweise und/oder zumindest teilweise unter Verwendung einer Bestrahlungsplanung (14, 15), insbesondere zumindest zeitweise und/oder zumindest teilweise unter Verwendung einer vorab erstellten und/oder einer im Verlauf der Bestrahlung erstellten und/oder angepassten Bestrahlungsplanung (14, 15) erfolgt.

9. Bestrahlungsvorrichtung (1) zur Bestrahlung zumindest eines Zielvolumenbereichs (3) eines Zielkörpers (2) mit einem Hadronenstrahl, umfassend einen Steuerungsrechner zur Bereitstellung einer Steuereingabe (21), durch welche der zu bestrahlende Zielvolumenbereich (3) des Zielkörpers (2) verändert werden kann, (wobei
- eine bereits erfolgte vorherige Bestrahlung eines Zielvolumenbereiches bei der Erzeugung (20, 21) der Steuereingabe (21) berücksichtigt wird,
- ein Korrekturvektor bei der Erzeugung (20, 21) der Steuereingabe (21) mittels des Steuerungsrechners angewendet wird, und wobei der Korrekturvektor von den folgenden Parametern abhängt:
- aktueller Teilchenfluss, und
- Geschwindigkeit der Bewegung des Zielvolumenbereichs (3) oder eines Materialausschnittes (16) gegenüber den äußeren Raumkoordinaten, und
- zumindest einer zwischen der Steuereingabe und einer Reaktion der Bestrahlungsvorrichtung (1) liegende Verzögerungszeitspanne (Δt), die zumindest zeitweise und/oder zumindest bereichsweise berücksichtigt (30) wird, wobei die Anwendung des Korrekturvektors auf die Steuereingabe, und die Steuerung des Hadronenstrahls mittels der Steuereingabe sich so auswirkt, dass der Zielvolumenbereich angesteuert wird, von dem zu erwarten ist, dass er sich zum Zeitpunkt der tatsächlichen Bestrahlung dort befindet, wo die Strahlung der Bestrahlungsvorrichtung tatsächlich einwirkt.

10. Bestrahlungsvorrichtung (1) nach vorstehendem Anspruch,
wobei bei der Erzeugung der Steuereingabe eine Mehrzahl von Verzögerungszeitspannen (Δt) mit zumindest zeitweise und/oder zumindest teilweise unterschiedlicher und/oder variierender Länge berücksichtigt wird.

11. Bestrahlungsvorrichtung (1) nach einem der Ansprüche 10 oder 11, ferner umfassend eine Ablenkvorrichtung (10) zur Ablenkung des Hadronenstrahls in lateraler und/oder longitudinaler Richtung,
wobei mittels des Steuerungsrechners bei der Ansteuerung des Zielvolumenbereichs von dem Hadronenstrahl berücksichtigt wird, dass die zwischen der Steuereingabe und der Reaktion der Bestrahlungsvorrichtung (1) liegende Verzögerungszeitspanne (Δt) bei einer lateralen Ablenkung (x-y) des Hadronenstrahls mittels der Ablenkvorrichtung (10) im Vergleich zu einer longitudinalen Ablenkung (z) unterschiedlich groß ausfällt.

12. Bestrahlungsvorrichtung (1) nach einem der Ansprüche 10 bis 13, ferner umfassend
zumindest eine Bestrahlungs-Anpassvorrichtung (10, 11) sowie
zumindest eine Steuervorrichtung zur Steuerung des Hadronenstrahls, welche derart ausgebildet und eingerichtet ist, dass sie mittels der Bestrahlungs-Anpassvorrichtung (10, 11) ein Verfahren (18, 20) nach einem der Ansprüche 1 bis 9 durchführt.

## Claims

1. A method (18, 20) for operating an irradiation device (1) for irradiating at least one target volume region (3) of a non-living target body (2) with a hadron beam, wherein the target volume region (3) to be irradiated of the target body (2) can be modified by a control input (21) of a control computer of the irradiation device (1),
wherein in generating (20, 21) the control input (21)
- an already previously performed irradiation of a target volume region is taken into account; and
- a correction vector is applied by the control computer, wherein the correction vector depends on the following parameters:
- current particle flow; and
- rate of movement of the target volume region (3) or of a material section (16) relative to the external spatial coordinates; and
- at least one delay time interval (Δt) between the control input and a response of the irradiation device (1), which is taken into account (30) at least temporarily and/or at least locally; and
wherein the application of the correction vector to the control input and the controlling of the hadron beam by the control input causes the hadron beam to target the target volume region which is expected to be located where the radiation of the irradiation device actually acts at the time of actual irradiation,

2. The method (18, 20) according to claim 1, **characterized in that** a plurality of delay time intervals (Δt) of at least temporarily and/or at least partially different and/or varying lengths are taken into account.

3. The method (18,20) according to claim 1 or 2, wherein in generating the control input it is taken into account that when the hadron beam targets the target volume region, the delay time interval (Δt) between the control input and the response of the irradiation device (1) is different for a lateral deflection (x-y) of the hadron beam compared to a longitudinal deflection (z) thereof.

4. The method (18, 20) according to any one of the preceding claims, **characterized in that** in case of a lateral deflection (x-y) of the hadron beam the delay time interval (Δt) is in a range below 250 ms, preferably in a range below 100 ms; and/or that
in case of a longitudinal deflection (z) of the hadron beam the delay time interval (Δt) is in a range below 150 ms, preferably in a range below 80 ms.

5. The method (18,20) according to any one of the preceding claims, **characterized in that** at least one delay time interval (Δt) takes into account signal conditioning effects and/or signal propagation effects.

6. The method (18, 20) according to any one of the preceding claims, **characterized in that** a temporal prediction algorithm (17) is used at least temporarily and/or at least locally, wherein the temporal prediction algorithm is used to make a prediction about the further movement of the target body and/or of the target volume region based on the current movement of the target body and/or of the target volume region;
wherein the temporal prediction algorithm can be effected by linear extrapolation, cubic extrapolation, spline extrapolation, neural networks or Markov chains.

7. The method (18,20) according to any one of the preceding claims, **characterized in that** the irradiation is performed as a scanning method or as a raster scanning method (18) or else as an intensity-controlled raster scanning method (18), at least partially and/or at least locally.

8. The method (18,20) according to any one of the preceding claims, **characterized in that** the control input (21) is generated using irradiation planning (14,15), at least temporarily and/or at least partially, in particular using previously created irradiation planning and/or irradiation planning (14,15) created and/or adapted during the irradiation, at least temporarily and/or at least partially.

9. An irradiation device (1) for irradiating at least one target volume region (3) of a target body (2) with a hadron beam, comprising a control computer for providing a control input (21) which can be used to modify the target volume region (3) of the target body (2) to be irradiated; wherein
- an already previously performed irradiation of a target volume region is taken into account in the generation (20,21) of the control input (21);
- a correction vector is applied for the generation (20,21) of the control input (21) using the control computer; and
- wherein the correction vector depends on the following parameters:
- current particle flow; and
- rate of movement of the target volume region (3) or of a material section (16) relative to the external spatial coordinates; and
- at least one delay time interval (Δt) between the control input and a response of the irradiation device (1), which is taken into account (30) at least temporarily and/or at least locally;
wherein as a result of the application of the correction vector to the control input and the controlling of the hadron beam using the control input, the target volume region is targeted which is expected to be located where the radiation of the irradiation device actually acts at the time of the actual irradiation.

10. The irradiation device (1) according to the preceding claim, wherein in the generation of the control input, a plurality of delay time intervals (Δt) of at least temporarily and/or at least partially different and/or varying lengths are taken into account.

11. The irradiation device (1) according to any one of claims 10 or 11, further comprising a deflection device (10) for deflecting the hadron beam in lateral and/or longitudinal directions;
wherein in controlling the hadron beam to target the target volume region using the control computer it is taken into account that the delay time interval (Δt) between the control input and the response of the irradiation device (1) is different for a lateral deflection (x-y) of the hadron beam compared to a longitudinal deflection (z) thereof.

12. The irradiation device (1) according to any one of claims 10 to 13, further comprising at least one irradiation adaptation device (10,11); and
at least one control device for controlling the hadron beam, which is configured and adapted for performing a method (18,20) according to any one of claims 1 to 9 using the irradiation adaptation device (10,11).

## Revendications

1. Procédé (18, 20) permettant de faire fonctionner un dispositif d'irradiation (1) en vue de l'irradiation d'au moins une zone de volume cible (3) d'un corps cible (2) non vivant, avec un faisceau de hadrons, la zone de volume cible (3) du corps cible (2) pouvant être modifiée par une entrée de commande (21) d'un ordinateur de commande du dispositif d'irradiation (1),
selon lequel
lors de la génération (20, 21) de l'entrée de commande (21),
- une irradiation d'une zone de volume cible, effectuée précédemment, est prise en compte, et
- un vecteur de correction est appliqué au moyen de l'ordinateur de commande, sachant que le vecteur de correction dépend des paramètres suivants :
- flux de particules actuel, et
- vitesse du mouvement de la zone de volume cible (3) ou d'une portion de matière (16) par rapport aux coordonnées spatiales extérieures, et
- au moins un laps de temps de retard (Δt) qui est compris entre l'entrée de commande et une réaction du dispositif d'irradiation (1) et qui est pris en compte (30) au moins temporairement et/ou localement, et
selon lequel l'application du vecteur de correction à l'entrée de commande et la commande du faisceau de hadrons au moyen de l'entrée de commande a pour effet que la zone de volume cible est visée par le faisceau de hadrons pour lequel on peut s'attendre à ce que, à l'instant de l'irradiation effective, il se trouve à l'endroit où le rayonnement du dispositif d'irradiation agit effectivement.

2. Procédé (18, 20) selon la revendication 1, **caractérisé en ce que** l'on prend en compte une pluralité de laps de temps de retard (Δt) dont la longueur est différente et/ou varie au moins temporairement et/ou au moins partiellement.

3. Procédé (18, 20) selon la revendication 1 ou 2, selon lequel on prend en compte, lors de la génération de l'entrée de commande, que lorsque le faisceau de hadrons est dirigé sur la zone de volume cible, le laps de temps de retard (Δt) compris entre l'entrée de commande et la réaction du dispositif d'irradiation (1) est différent pour une déviation latérale (x-y) du faisceau de hadrons par rapport à une déviation longitudinale (z).

4. Procédé (18, 20) selon une des revendications précédentes, **caractérisé en ce que** pour une déviation latérale (x-y) du faisceau de hadrons, le laps de temps de retard (Δt) se situe dans une plage inférieure à 250 ms, de préférence dans une plage inférieure à 100 ms, et/ou **en ce que** pour une déviation longitudinale (z) du faisceau de hadrons, le laps de temps de retard (Δt) se situe dans une plage inférieure à 150 ms, de préférence dans une plage inférieure à 80 ms.

5. Procédé (18, 20) selon une des revendications précédentes, **caractérisé en ce qu'**au moins un laps de temps de retard (Δt) prend en compte des effets de traitement de signaux et/ou des effets de transmission de signaux.

6. Procédé (18, 20) selon une des revendications précédentes, **caractérisé en ce que** l'on utilise au moins temporairement et/ou au moins localement un algorithme de prédiction temporelle (17), sachant que l'on utilise l'algorithme de prédiction temporelle pour faire une prédiction, sur la base du mouvement actuel du corps cible et/ou de la zone de volume cible, concernant la suite du mouvement du corps cible et/ou de la zone de volume cible,
sachant que l'algorithme de prédiction temporelle peut être exécuté par extrapolation linéaire, extrapolation cubique, extrapolation de splines, par des réseaux neuronaux ou par des chaînes de Markov.

7. Procédé (18, 20) selon une des revendications précédentes, **caractérisé en ce que** l'irradiation est réalisée au moins partiellement et/ou au moins localement comme procédé de balayage ou comme procédé de balayage trame (18), ou bien comme procédé de balayage trame (18) à commandé d'intensité.

8. Procédé (18, 20) selon une des revendications précédentes, **caractérisé en ce que** la génération de l'entrée de commande (21) est réalisée au moins temporairement et/ou au moins partiellement en utilisant un plan d'irradiation (14, 15), en particulier au moins temporairement et/ou au moins partiellement en utilisant un plan d'irradiation (14, 15) établi préalablement et/ou établi au cours de l'irradiation et/ou adapté.

9. Dispositif d'irradiation (1) destiné à l'irradiation d'au moine une zone de volume cible (3) d'un corps cible (2) avec un faisceau de hadrons, comprenant un ordinateur de commande destiné à fournir une entrée de commande (21) permettant de modifier la zone de volume cible (3) du corps cible (2) devant être irradiée,
dans lequel
- une irradiation d'une zone de volume cible, effectuée précédemment, est prise en compte lors de la génération (20, 21) de l'entrée de commande (21),
- un vecteur de correction est utilisé lors de la génération (20, 21) de l'entrée de commande (21) au moyen de l'ordinateur de commande, et
dans lequel le vecteur de correction dépend des paramètres suivants :
- flux de particules actuel, et
- vitesse du mouvement de la zone de volume cible (3) ou d'une portion de matière (16) par rapport aux coordonnées spatiales extérieures, et
- au moins un laps de temps de retard (Δt) qui est compris entre l'entrée de commande et une réaction du dispositif d'irradiation (1) et qui est pris en compte (30) au moins temporairement et/ou localement,
sachant que l'application du vecteur de correction à l'entrée de commande et la commande du faisceau de hadrons au moyen de l'entrée de commande a pour effet que la zone de volume cible est visée, pour laquelle on peut s'attendre à ce que, à l'instant de l'irradiation effective, elle se trouve à l'endroit où le rayonnement du dispositif d'irradiation agit effectivement.

10. Dispositif d'irradiation (1) selon la revendication précédente, dans lequel, lors de la génération de l'entrée de commande, on prend en compte une pluralité de laps de temps de retard (Δt) dont la longueur est différente et/ou varie au moins temporairement et/ou au moins partiellement.

11. Dispositif d'irradiation (1) selon une des revendications 10 ou 11, comprenant en outre un dispositif de déviation (10) destiné à dévier le faisceau de hadrons dans la direction latérale et/ou longitudinale, sachant que l'on prend en compte, au moyen de l'ordinateur de commande, lorsque le faisceau de hadrons est dirigé sur la zone de volume cible, que le laps de temps de retard (Δt) compris entre l'entrée de commande et la réaction du dispositif d'irradiation (1) est différent pour une déviation latérale (x-y) du faisceau de hadrons par rapport à une déviation longitudinale (z).

12. Dispositif d'irradiation (1) selon une des revendications 10 à 13, comprenant en outre au moins un dispositif d'adaptation d'irradiation (10, 11) ainsi qu'au moins un dispositif de commande qui est destiné à commander le faisceau de hadrons et qui est réalisé et conçu de manière à mettre en oeuvre, au moyen du dispositif d'adaptation d'irradiation (10, 11), un procédé (18, 20) selon une des revendications 1 à 9.
